# EUROPEAN PATENT APPLICATION

(11) **EP 2 327 440 A1**
(43) Date of publication of application: **01.06.2011**
(21) Application number: 09812729.3
(22) Date of filing: 28.08.2009
(51) Int. Cl.: A61M 16/04, A61B 1/24

(54) **IMPROVED MOUTH OPENER FOR INTRODUCING LARYNGEAL MASKS AND OTHER MEDICAL DEVICES THROUGH THE ORAL CAVITY**

(30) Priority: 10.09.2008 ES 200802581
(71) Applicant: Fundació Hospital Asil de Granollers, 08402 Granollers (Barcelona) (ES)
(72) Inventor: POCH MARTI, Pere, 08397 PINEDA DE MAR ( Barcelona) (ES)
(74) Representative: Torner Lasalle, Elisabet
(86) International application number: PCT/ES2009/070358
(87) International publication number: WO 2010/029202

(57) **Abstract**

The invention relates to a tool which allows solving the existing drawbacks when operating in the buccal cavity and introducing other devices, as well as the need of previously mounting the laryngeal mask system on the components of said introductory tool and requiring the assistance of an auxiliary person to stabilize the head of the patient. Firstly, the tool acts as a mouth gag, and secondly it acts as a tool to facilitate the introduction of the laryngeal mask and is formed by an upper or palatine intraoral portion, a lower or lingual intraoral portion, a mechanism for separating the two intraoral portions, a self-locking mechanism of the lower portion and an unlocking mechanism of the aforementioned self-locking mechanism.

## Description

### Object of the Invention

The invention more specifically relates to improvements introduced in a tool used in operating rooms, the purpose of which is to introduce laryngeal masks when performing surgery on a patient and it is necessary to be able to operate within the buccal cavity of said patient without him unexpectedly closing his mouth in a reflex action, said improvements being oriented towards modifying said tool so that it serves at the same time as a mouth gag and a guiding means for the introduction of laryngeal masks, as well as other medical devices through the oral cavity.

### State of the Art

Tools known as mouth gags, the purpose of which is the application thereof to the mouth of the patient who is to undergo a surgical operation and whose mouth must be kept open to be able to introduce other tools inside the buccal cavity or to access other pathways such as the respiratory or digestive pathways, can be found on the market and can therefore be considered as state of the art.

Tools of another type facilitate in the way that they have been designed the introduction of laryngeal masks or other medical devices, with or without a mouth gag, once the mouth is open, making it necessary to have a second auxiliary person to press the forehead of the patient or to pull on his chin, and to prevent the patient from instinctively lowering his head when the tool is introduced, complicating the operation for introducing the tool; an example of this type is Spanish patent no. 2,322,616, of the same proprietor, which allows opening the mouth by means of continuous pulling with the introductory tool.

Another embodiment of the same type of tool is that shown in patent WO 2007087446 which prevents tongue obstruction when a laryngeal mask is introduced and acts by exerting a lingual pressure, similar to the aforementioned ES patent no. 2,332,616.

Another embodiment of the same type of tool is that shown in patents US 2004060564 and GB 2,436,294 which facilitates the introduction of the laryngeal mask when it reaches the rear part of the mouth, forcing it to curve with the help of a rigid curved tube.

However, the embodiments contemplated in these and other patents forming the state of the art have a series of drawbacks such as:
- They require previously mounting the laryngeal mask in one of the components of the introductory tool.
- They require the mounting of laryngeal mask system with the second component of the introductory tool.
- They require the assistance of an auxiliary person to stabilize the head of the patient when pulling the jaw downwards with the introductory tool.

### Purpose of the Invention

To solve the drawbacks pointed out above such that the following advantages are achieved:
- An auxiliary person to open the mouth and subsequently introduce the laryngeal mask is not required.
- The additional preparation of the laryngeal mask is not necessary, only the usual lubrication is.
- Once the mouth is open, it remains open without requiring the continuous assistance of the person who places the laryngeal mask, freeing up one hand, such that it facilitates the manipulation thereof.
- The tongue of the patient remains in the lowest possible plane, conferring maximum space to the buccal cavity for working.
- The palatine portion of the mouth gag directs the laryngeal mask towards the hypopharynx.
- The improved mouth gag facilitates the introduction of other medical devices in addition to the aforementioned masks, such devices can be devices for controlling the airway, fiberscopes, gastroscopes, secretion aspirators and others.
- In the open position, it facilitates the permeability of the airway and the patient's breathing.
- In the open position, it prevents the patient from possibly biting, crushing the medical device that passes through the inside of the mouth gag.

### Description of the Invention

The objective of the present is a tool firstly acting as a mouth gag, and secondly as a tool to facilitate the introduction of the laryngeal mask once the patient opens his mouth as a result of the introduction of the tool which is referred to as a mouth gag in the buccal cavity.

For the purposes of the invention, the buccal cavity is limited in the front by the lips and front teeth and in the rear by the oropharynx and hypopharynx, at the top by the palate with its two areas (the hard front palate and the soft rear palate), and at the bottom by the tongue. The passageways known as the esophagus and trachea start from the hypopharynx.

The mouth gag is formed by the following parts:
- Upper or palatine intraoral portion.
- Lower or lingual intraoral portion.
- Mechanism for separating the two intraoral portions.
- Self-locking mechanism of the lower portion.
- Unlocking mechanism of the above self-locking mechanism.

Said tool comprises a body formed by a considerably flat plate having a rectangular perimeter the central part of which has been provided with a large hole, and an also rectangular prolongation emerges from the lower base of the body of the plate in the form of a handgrip of the tool.

A second also flat plate screwed onto the handgrip has been provided in the handgrip, and it works as a means for guiding the lower or lingual intraoral portion, forming part of the self-locking mechanism as it is in contact with the tongue in the working position so that it can move up or down in relation to the body of the main plate in which the upper base of which is integral with an upper or palatine intraoral portion as it is with the palate in the working position.

In other words, the distance between the upper and lower portion can be regulated at will by the anesthesiologist so that it is fixed perfectly to the mouth of the patient and adapts to the geometry of the buccal cavity of said patient based on the premise that not all buccal cavities have the same geometry.

The handgrip together with the plate integral therewith form a mechanism for separating the two previously mentioned intraoral portions with the aid of said upper and lower portions.

The upper portion is configured according to a preferably concave profile an end of which is integral with the upper part of the body of the plate, whereas the lower portion considerably equal to the upper portion is integral with a guide that moves between the two previously described plates. A notched area that works together with a trigger allowing the guide to move in vertical direction and its immobilization in the suitable position, forming the locking and unlocking mechanism, has been provided in the guide.

When the guide moves up and down the trigger follows the notched area, whereas to move the guide upwards, the trigger must be moved outwards to allow the upward movement.

Other details and features will be shown in the course of the description which is given below referring to the drawings accompanying this specification, in which the preferred details of the invention are depicted in an illustrative and non-limiting manner.

### Description of the Drawings

Figure 1 is a schematic sectional view of the human head and more specifically the oral cavity and its surroundings.
Figure 2 is a schematic sectional view of the human head and more specifically the buccal cavity with the intraoral portion of the proposed mouth gag in the open position in the oral cavity oral when the laryngeal mask is introduced.
Figure 3 is a schematic sectional view of the human head and more specifically the oral cavity with the intraoral portion of the proposed mouth gag in the open position with the laryngeal mask completely introduced.
Figure 4 is an upper plan view of a conventional laryngeal mask.
Figure 5 is a lower plan view of a conventional laryngeal mask.
Figure 6 is a plan view of the body of the mouth gag.
Figure 7 is an elevated side view of the mouth gag in the open position.

### Description of a Preferred Embodiment of the Invention

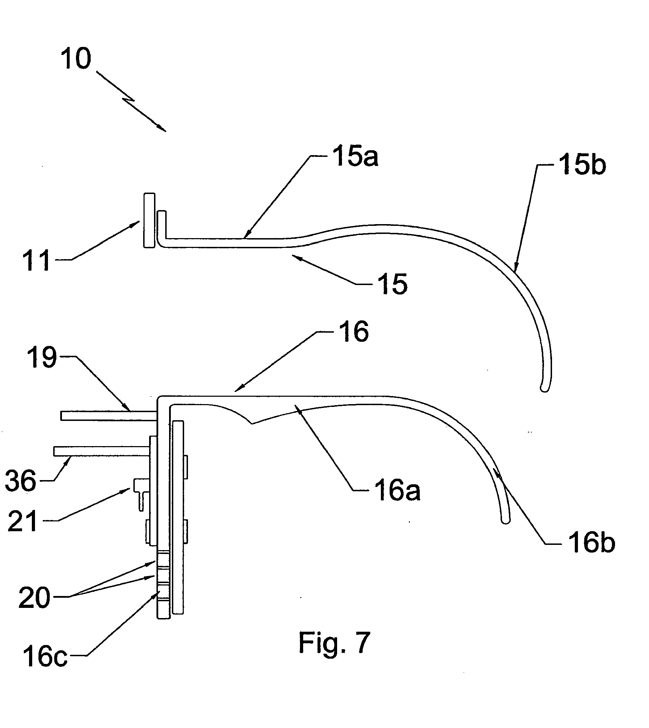

As can be seen in Figure 7, in one of its possible embodiments the proposed invention is formed by a flat plate (10), the body (11) of which has a considerably rectangular perimeter, the lower base (12) of which prolongs into a handgrip (13) having a rectangular perimeter on which a small plate (14) is made integral therewith by any known method. The central part of the plate (10) has a large opening (17), and a small recess (18) in the central part of the lower base (12). The plate (14) incorporates two pivots (36) perpendicular to the plate (14).

The plate (10) acts as means for supporting two parts, an upper part referred to as upper or palatine intraoral portion (15), and a lower intraoral portion (16) referred to as lingual portion. The upper portion (15), also see Figure 7, is integral with the plate (10) and with its body (11), whereas the lower portion (16) passes between the handgrip (13) and the plate (14).

The upper part (15) has a configuration with a flat first part (15 a) which prolongs into a considerably concave curved second part (15b) facing downwards, whereas the lower portion (16) is considerably flat at one of its ends (16a), whereas at the opposite end (16b) it is concave and facing downwards. Said lower portion (16) is bent downwards at one of its ends in a right angle according to a flat vertical part (16c) which works as a guide between the plate (14) and the handgrip (13) of the plate (10).

To facilitate the movement of the lower portion (16) with respect to the upper portion (15), the portion (16) incorporates a pivot (19) perpendicular to the vertical part (16c) or guide. A notched area in the form of a rack (20) working with a trigger (21) through a mechanism known as a ratchet mechanism, which is not described as it is technically conventional, has been provided in the vertical portion (16c) or guide as a safety element and to fix the relative position of (16) with respect to (15).

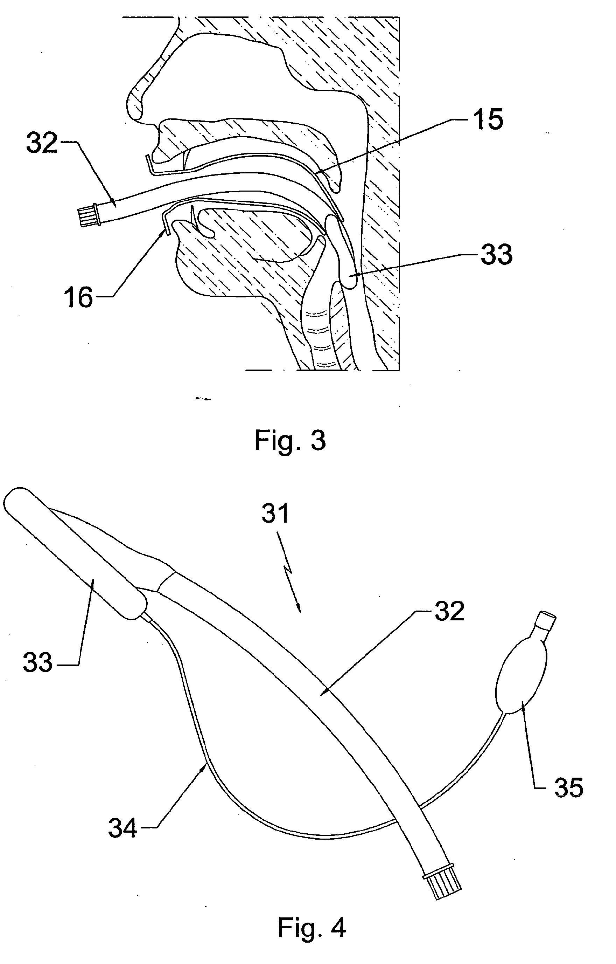

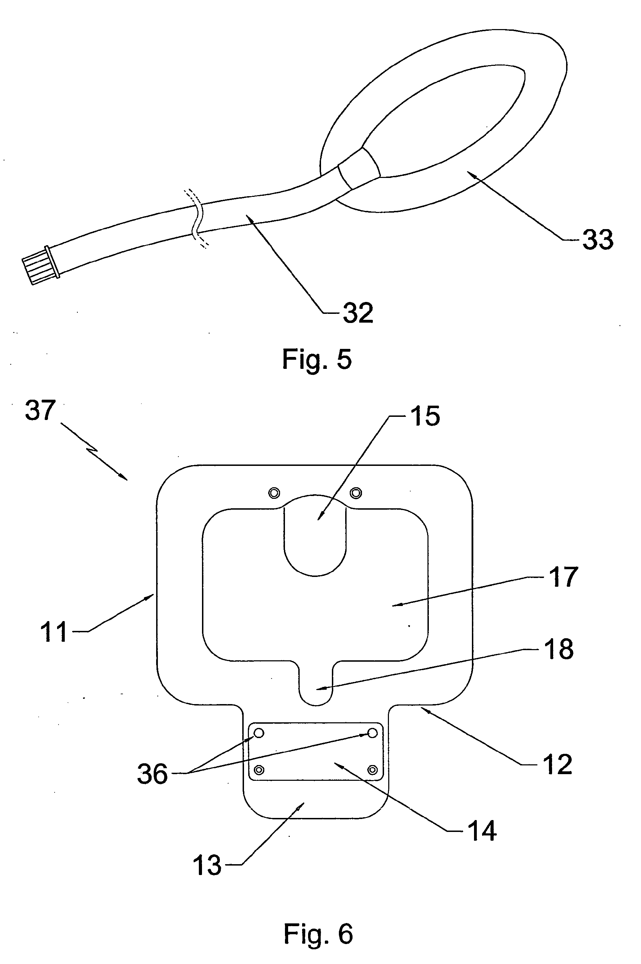

As shown in Figures 4 and 5, a laryngeal mask is formed by a tube (32) an end of which ends in part (33) which is the part that is coupled to the larynx of the patient, as illustrated in Figure 3.

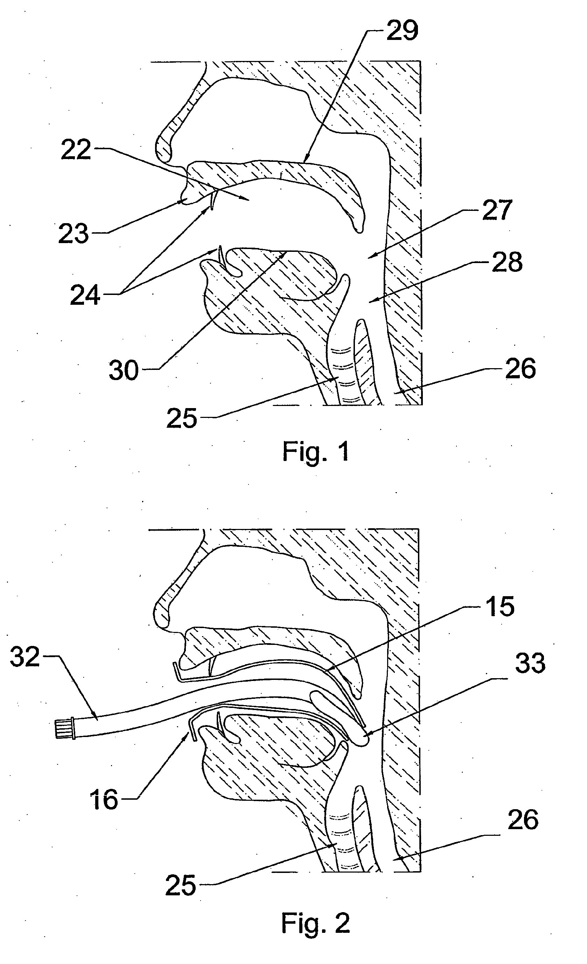

The mouth gag (10) operates when the anesthesiologist grabs it in the closed position-i.e., with the lingual portion (16) in contact with the palatine portion (15), with one hand on the handgrip (13), introducing it into the oral cavity (22) demarcated at the top by the palate (29) and at the bottom by the tongue (30), in the front part by the lips (23) and the line of teeth (24), and in the rear part by the oropharynx (27) and the hypopharynx (28). By means of applying a force using the thumb and index finger on the pivots (36) of the plate (14) and the pivot (19) of the portion (16), the mouth gag goes from the closed to the open position, the upper portion (15) being separated from the lower portion (16) as shown in Figure 2, being maintained in the open position by the self-locking mechanism (20).

The upper portion (15) is supported on the palate (29), whereas the lower portion (16) finds the surface of the tongue (30), pressing it downwards, the laryngeal mask (31) the formal features of which can be seen in Figure 4, being subsequently placed such that its end part (33) is introduced, sliding it by the upper portion (15) due to the fact that its concavity forces said end part (33) to follow a curvilinear path in accordance with the curvature of (15), and in turn being slid by the lower portion (16), forcing a subsequent pushing movement by the anesthesiologist with the hand on the tube (32) to place the aforementioned end part facing the trachea (25) and covering the esophagus (26), as seen in Figure 3.

The nature of the mouth gag (10) as a result of the possibility of regulating the distance between the upper portion (15) and the lower portion (16) allows it to be adapted to the geometry of each patient not only in terms of the height of the palate but also the curvature of the palate (29) in relation to the curvature of the oropharynx (27) and hypopharynx (28), facilitating the end part not getting stuck in the area (27) and the patient closing his mouth in a reflex movement such that the anesthesiologist loses sight of the cavity oral (22).

The removal of the improved mouth gag from the oral cavity (22) is facilitated by means of unlocking the portion (16) using the trigger (21) and the gradual removal of the intraoral portion (15 and 16) between the lips (23).

Having sufficiently described the present invention in correspondence with the attached drawings, it is readily understood that any modifications in detail that are considered appropriate can be introduced therein provided that the essence of invention summarized in the following claims is not changed.

## Claims

1. An improved mouth gag for the introduction of laryngeal masks and other medical devices through the oral cavity of the type formed by an angled blade made from a preferably stainless steel plate, the body of which is angular and with an end part in the form of a handle or grip which is used in operating rooms as a tool for separating the tongue from the palate of a patient and for leaving the buccal cavity free for the introduction of an anesthesia mask, mounted at the end of a tube, into the mouth of said patient for the purpose of getting it to the larynx, facing the opening of the trachea;
**characterized in that** the mouth gag (10) comprises:
- A flat plate (37) integral with an upper intraoral portion (15) to which the lower portion (16) and the locking and unlocking mechanisms are incorporated.
- An upper or palatine intraoral portion (15) integral with a plate (11).
- A lower or lingual intraoral portion (16) integral with a guide (16c).
- A mechanism for separating the two intraoral portions (15-16).
- A self-locking mechanism of the lower portion (16).
- An unlocking mechanism of aforementioned self-locking mechanism.

2. The improved mouth gag for the introduction of laryngeal masks and other medical devices through the oral cavity according to claim 1, **characterized in that** the flat plate (37) has a body (11), the lower base (12) of which prolongs into a handgrip (13) on which a small plate (14) is integral therewith, which incorporates two pivots (36) perpendicular to the aforementioned plate (14), a large opening (17) and a small recess (18) have been provided in the central part of the lower base (12).

3. The improved mouth gag for the introduction of laryngeal masks and other medical devices through the oral cavity according to claim 1, **characterized in that** the upper part (15) has a configuration with a considerably flat first part (15a) which prolongs into a considerably concave curved second part (15b) facing downwards.

4. The improved mouth gag for the introduction of laryngeal masks and other medical devices through the oral cavity according to claim 1, **characterized in that** the lower portion (16) is considerably flat at one of its ends (16a), whereas at the opposite end (16b) it is concave and faces downwards, being bent downwards in a right angle at one of its ends according to a flat vertical part which passes between the plate (14) and the handgrip (13) of the plate (37), incorporating a pivot (19) perpendicular to the vertical part (16c).

5. The improved mouth gag for the introduction of laryngeal masks and other medical devices through the oral cavity according to claim 1, **characterized in that** the self-locking mechanism of the guide (16c) includes in the vertical portion (16c) a notched area in the form of a rack (20) working with a trigger (21) located on the plate (14) to fix the relative position of the upper portion (15) with respect to the lower portion (16).

6. The improved mouth gag for the introduction of laryngeal masks and other medical devices through the oral cavity according to claim 1, **characterized in that** the unlocking mechanism of the guide (16c) is operated upon moving the trigger (21) located on the plate (14) outwards to allow the upward movement of said guide (16c).

## Amended claims

### Amended claims under Art. 19.1 PCT

**1.** An improved mouth gag for the introduction of laryngeal masks and other medical devices through the oral cavity of the type formed by an angled blade made from a preferably stainless steel plate, the body of which is angular and with an end part in the form of a handle or grip, **characterized in that** the mouth gag (10) comprises:
- A flat plate (37) with a large opening on its central part (17), integral with an upper or palatine intraoral portion (15) to which the lower or lingual (16) and the locking and unlocking mechanisms are incorporated.
- A lower or lingual intraoral portion (16) bent downwards at one of its ends in a right angle.
- A mechanism for separating the two intraoral portions (15-16).
- A self-locking mechanism of the lower or lingual intraoral portion (16).
- An unlocking mechanism of aforementioned self-locking mechanism.

**2.** The improved mouth gag for the introduction of laryngeal masks and other medical devices through the oral cavity according to claim 1, **characterized in that** the flat plate (37) has a body (11), the lower base (12) of which prolongs into a handgrip (13) on which a small plate (14) is integral therewith, which incorporates two pivots (36) perpendicular to the aforementioned plate (14), a large opening (17) and a small recess (18) have been provided in the central part of the lower base (12).

**3.** The improved mouth gag for the introduction of laryngeal masks and other medical devices through the oral cavity according to claim 1, **characterized in that** the upper or palatine intraoral portion (15) has a configuration with a considerably flat first part (15a) which prolongs into a considerably concave curved second part (15b) facing downwards.

**4.** The improved mouth gag for the introduction of laryngeal masks and other medical devices through the oral cavity according to claim 1, **characterized in that** the lower or lingual intraoral portion (16) is considerably flat at one of its ends (16a), whereas at the opposite end (16b) it is concave and faces downward, being bent downwards in a right angle at one of its ends according to a flat vertical part which passes between the plate (14) and the handgrip (13) of the plate (37), incorporating a pivot (19) perpendicular to the vertical part (16c).

**5.** The improved mouth gag for the introduction of laryngeal masks and other medical devices through the oral cavity according to claim 1, **characterized in that** the self-locking mechanism of the lower or lingual intraoral portion (16) includes a notched shape in the form of a rack (20) working with a trigger (21) located on the plate (14) to fix the relative position of the upper or palatine intraoral portion (15) with respect to the lower or lingual intraoral portion (16).

**6.** The improved mouth gag for the introduction of laryngeal masks and other medical devices through the oral cavity according to claim 1, **characterized in that** the unlocking mechanism of the lower or lingual intraoral portion (16c) is operated upon moving the trigger (21) located on the plate (14) outwards to allow the upward movement of the lower or lingual intraoral portion (16).
